# EUROPEAN PATENT APPLICATION

(11) **EP 4 574 014 A1**
(43) Date of publication of application: **25.06.2025**
(21) Application number: 23218067.9
(22) Date of filing: 19.12.2023
(51) Int. Cl.: A61B 1/06, A61B 1/00

(54) **ENDOSCOPE WITH LIGHT GUIDE AND LEDS IN DISTAL TIP**

(71) Applicant: Ambu A/S, 2750 Ballerup (DK)
(72) Inventor: SØRENSEN, Morten, 2750 Ballerup (DK)
(74) Representative: Winter, Brandl - Partnerschaft mbB

(57) **Abstract**

An endoscope (1) with a distal tip (10), where the distal tip comprises a camera (11) and a light emitter (13). The light emitter comprises a first LED (15) configured to emit light with a first spectral distribution, and a second LED (15') configured to emit light with a second spectral distribution, different from the first spectral distribution. The light emitter comprises a light guide (30) with a length, and a width transverse to the length. The light guide comprises a light entry end (31) and a light exit (32) end configured such that light passes the light guide in the length direction. The light emitter also comprises a diffusor (34). The first and the second LEDs are arranged to emit light into the light entry end, and the diffusor is arranged such that light passing the light exit end also passes through the diffusor.

## Description

### Technical Field

The present teaching relates to an endoscope comprising a light emitter in a distal tip. The light emitter comprises diodes having different spectral distributions and a light guide.

### Background

In endoscopy for medical purposes endoscopes are used for visual navigation into, and examination and diagnosis of, hollow organs and body cavities. For such purposes the quality of the image provided to the endoscope user is an important parameter. The level of this quality is primarily influenced by the camera applied and by the light provided to the target to be observed. Both the amount of light and the distribution of light in the image field of view is important for the image quality.

When applying endoscopes in medical procedures it is, for some procedures, preferred to have the option of changing the spectral distribution of the light during the procedure. This is typically the case when specific types of tissue or characteristics in the tissue are to be enhanced. Such image enhancement can be done by different application of fluorescence techniques, or by application of light having a spectral distribution with peaks at wavelengths where tissue or cells are e.g., known to have a high absorbance. For example, it is possible to enhance image contrast of the vascular areas relative to surroundings, by providing peaks of light intensity at wavelengths where hemoglobin has a high absorbance.

To enable changing between e.g., a white light mode and a mode with colored light having peaks at specific wavelengths, it is necessary to be able to change between different spectral distributions of the light during a procedure. This can be done by having at least two different light sources available and being able to turn these on and off separately. The light sources may be arranged in a special box connected to the endoscope and the light emitted from the light sources guided via optical fibers through the endoscope to the distal tip thereof.

For single-use endoscopes, i.e., designed to be used for only one procedure and then discarded, it may be preferred to have the light source built into the distal tip of the endoscope, whereby optical fibers going from the handle of the endoscope to the distal tip can be avoided, thereby facilitating a simpler and cheaper manufacturing.

A previous approach can be found in JP 11-267099 A2.

When viewing tissue with an endoscope, direct reflections of the light source will often be visible in the image, especially when surfaces with a high specular reflectance, such as humid or wet surfaces are viewed. Users of endoscopes will be familiar with this and will ignore it. However, if the white light is formed from two or more light sources having different colors, there may be a separate reflection of each light source having the color of this light source. This may be distracting for the endoscope user.

### Summary

The present teaching provides an endoscope comprising a distal tip having a light emitter and a camera, where the light emitter comprises, Light Emitting Diodes (LEDs) having different spectral distributions and a light guide.

In one embodiment, the present teaching relates to an endoscope with a distal tip, where the distal tip comprises a camera with an optical axis, and a light emitter. The light emitter comprises a first LED configured to emit light with a first spectral distribution, and a second LED configured to emit light with a second spectral distribution which is different from the first spectral distribution. Further, the light emitter comprises a light guide with a length, and a width transverse to the length, the light guide comprises a light entry end and a light exit end configured such that light passes the light guide in the length direction. The light emitter also comprises a diffusor arranged transverse to the optical axis. The first and the second LED are arranged to emit light into the light entry end, and the diffusor is arranged such that light passing the light exit end also passes the diffusor. The length direction may be parallel to the optical axis.

It has been found that this embodiment can provide a better mixing of different colored light into one combined spectrum, and thereby reduce colored reflections, considerably, or even eliminate such reflections. This is achieved by an improved mixing of light from the first LED and the second LED.

Also, the embodiment achieves a very homogenous distribution of light, such that the image quality of the endoscope is improved.

In a second aspect the teaching relates to a system comprising an endoscope according to the present embodiment and variations thereof, a monitor and a control unit.

Further features and combinations consistent with the present teachings will become apparent from the dependent claims and from the following detailed description. Features described in the present teaching can be combined so as to form further arrangements within the scope of the present teaching that are not explicitly set out herein.

In this teaching, the expression "distal" is defined to be in the direction away from the user of the endoscope and toward the patient, and "proximal" is defined to be closest to the endoscope user. For the handle of the endoscope, the distal end will be the end where the main tube is connected, and the proximal end is the opposite end. Further, the expression "handle" may be a positioning interface, or interface, which functions to control the position of the insertion cord. The handle, or positioning interface, may be an interface operated by a robotic arm, or it may be a handle operated by the hand of an endoscope user.

### Brief description of the figures

The above-mentioned embodiments, features and advantages thereof will be further elucidated by the following illustrative and nonlimiting detailed description of embodiments disclosed herein with reference to the appended drawings, wherein:
Fig. 1 shows a visualization system comprising an endoscope and a monitor with a control unit.
Fig. 2 shows a distal tip housing seen from a distal end.
Fig. 3 shows a distal tip housing connected to a bending section.
Fig. 4 shows schematic view of a camera.
Fig. 5 shows a distal tip housing seen from a proximal end.
Fig. 6 shows a perspective view of a light emitter.
Fig. 7 illustrates the intensity distribution of light exiting a light guide having a length of 1 mm.
Fig. 8 illustrates the intensity distribution of light exiting a light guide having a length of 2 mm.
Fig. 9 illustrates the intensity distribution of light exiting a light guide having a length of 4 mm.
Fig. 10 illustrates the intensity distribution of light exiting a light guide having a length of 6 mm.

In the drawings, corresponding reference characters indicate corresponding parts, functions, and features throughout the several views. The drawings are not necessarily to scale and certain features may be exaggerated in order to better illustrate and explain the disclosed embodiments. For simplicity, this teaching will focus on a two-way bending endoscope, but the teaching is relevant for, and covers, also a four-way bending endoscope.

### Detailed description

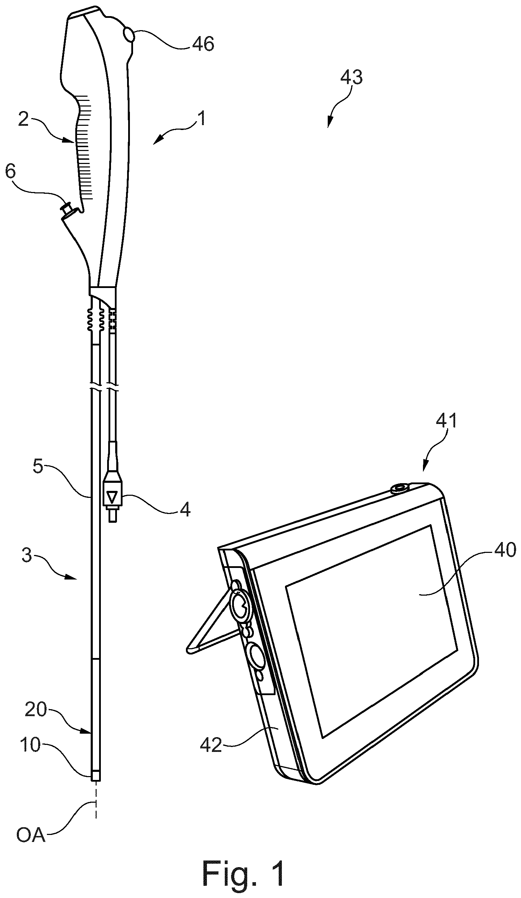

Fig. 1 illustrates a visualization system 43 comprising an endoscope 1 and a monitor 41. The endoscope comprises a handle 2, an insertion cord 3 and an electrical cable with a connector 4 for connecting the endoscope 1 to the monitor 41. The insertion cord 3 is the part to be inserted into a body lumen during an endoscopic procedure. The insertion cord 3 comprises a distal tip 10, a bending section 20 and a main tube 5. The handle 2 may comprise an entrance to a working channel 6 running through the insertion cord 3 to an opening at the distal tip 10. The handle also comprises a bending lever 46, which can be used for bending the bending section 20.

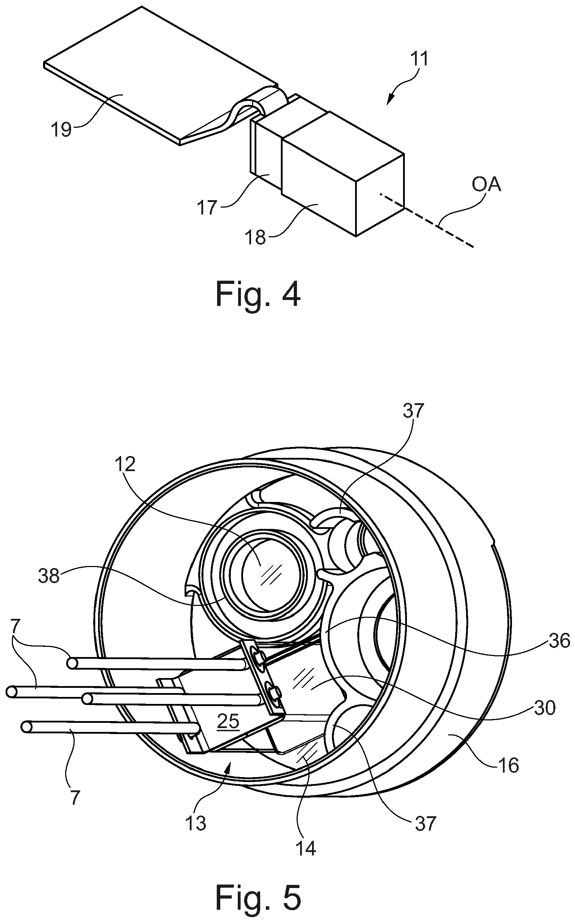

The monitor 41 comprises a screen 40 and a control unit 42. The control unit 42 comprises an electronic circuit for receiving and processing the image stream from the camera 11 (see fig. 4) as well as a processor for image processing, user interface, storage of images etc. The screen 40 displays the image stream from the camera 11. The monitor 41 may be one unit or housing comprising both screen 40 and control unit 42. The screen 40 and the control unit 42 may also be separate parts, e.g., two separate units or housings (not shown).

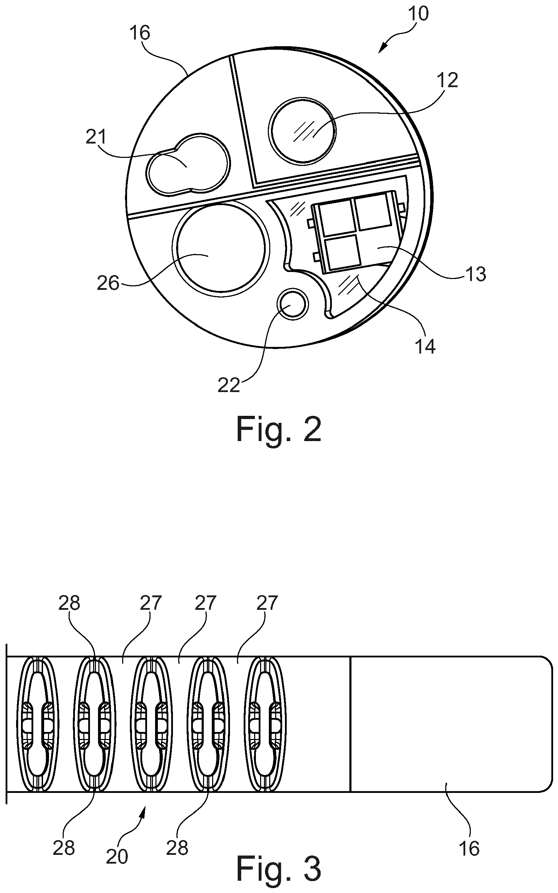

Fig. 2 shows an example of a distal tip 10 of the endoscope 1 in fig. 1 viewed from a distal end. The distal tip 10 comprises tip housing 16 including a camera window 12, a light emitter window 14, a cleaning nozzle 21, and a distal working channel opening 26. Instruments can be applied e.g., for taking tissue samples, through the distal working channel opening 26. The cleaning nozzle 21 is applied for cleaning the outside of the camera window 12 by irrigation of cleansing water. The waterjet nozzle 22 is provided for possible cleaning of any tissue to be studied by use of the endoscope. A light emitter 13 is shown, positioned proximally of the light emitter window 14, and is described below.

As shown in Fig. 3, the distal tip house 16 may be connected to a distal end of the bending section 20. The bending section 20 may be molded in one piece from a polymer material, such as Polyoxymethylene (POM). Typically, a bending cover (not shown) is covering the bending section 20 to make this part watertight and to provide a smooth outer surface.

The bending section is attached to the main tube 5 in the proximal end. In this example the bending section is molded in one piece and comprises a number of segments 27. The proximal end segment 27 is connected to the distal end of the main tube 5 (not shown in Fig. 3). The segments are held together by hinges 28, so that the segments can be bent relative to each other by manipulation of steering wires (not shown) controlled by the bending lever 46. In this example, the hinges 28 are integrally molded with the segments 27 to form a one-piece bending section 20.

Referring now to Fig 4, the distal tip 10 comprises a camera 11 having an image sensor 17 and a lens stack 18. The camera 11 is placed proximally and adjacent the camera window 12, and arranged to view e.g., tissue on the external side of the endoscope distal tip or any observation target which is found relevant to study during a procedure. The camera 11 has an optical axis OA which may be coincident with a center axis of the lens stack 18. The optical axis OA may extend perpendicular to a plane formed by the image sensor 17.

The camera 11 will have a field of view, measured as an angle, defined by the image sensor 17 and the lens stack 18. Images or a stream of images captured by the camera 11 may be shown on the monitor 41, e.g., after image processing.

Often, the camera 11, or part of it, is arranged inside a distal tip housing 16 (which is illustrated in fig. 5). The camera 11 may comprise a flexible printed circuit board 19 connecting the image sensor 17 to wires (not shown) which are connected to a circuit board in the handle or to the control unit 42. A camera frame (not shown) made of a polymer material may support the flexible printed circuit board 19. The lens stack 18 of the camera 11 defines the optical axis OA, which may extend through a center of the image sensor 17 and a center of the imaged part of the target to be observed. The optical axis may extend perpendicular to the image sensor 17, and through a center of the image sensor 17. The center of the image sensor 17 may here be defined as the center of the part of the image sensor 17 forming the image to be analyzed and presented to a user of the endoscope 1. The optical axis OA may extend in parallel with a longitudinal axis extending in a distal to proximal direction of the distal tip 10.

Fig. 5 shows the distal tip housing 16 viewed from a proximal end in a perspective view. The distal tip housing 16 may be molded from a polymer material, e.g., from a thermoplastic polymer, such as polycarbonate (PC) or COC, COP, PMMA. Also, silicone may be applied for the distal tip housing. The distal tip housing 16 may be molded in a two-component molding process, from a transparent material and a non-transparent material, respectively. The transparent material may preferably be applied for the camera window 12 and light emitter window 14. The non-transparent material may be applied for the rest of the distal tip housing 16. By applying the two-component molding process, the transparent and non-transparent parts of the housing will be fused together in a strong and watertight connection. Also, a process of connecting two separate housing parts, e.g., by gluing, can be avoided. This may simplify manufacturing. Also, the application of non-transparent material for parts of the housing, other than the windows, reduces the risk of stray-light into the camera.

Inside the distal tip housing 16, internal structures may be provided. Such internal structures are seen in fig. 5 and may be molded as part of the distal tip housing 16. This internal structure may comprise a support wall 36 for the working channel tubing and support walls 37 for the media tubes providing water for the cleaning nozzle 21 or waterjet nozzle 22. The internal structure may further comprise support structure 38 for supporting and aligning the camera 11. Such support structure 38 for the camera may be a wall encircling the distal part of the camera. Such a wall made from a non-transparent material, may support the positioning of the camera 11 and may also prevent stray-light from an LED, potentially an LED of the light emitter, passing into the camera. Support walls 36, 37 and support structure 38 enable a simple and fast assembly of the distal tip 10 providing stable and watertight connections.

In an example, the distal tip comprises a housing 16 with a support 38 for positioning the camera 11, where the support 38 is non-transparent to prevent stray-light into the camera 11 and image sensor 17. As any stray-light could originate from one LED alone, this feature further reduces the risk of having parts of the image from the endoscope unintentionally colored.

As illustrated in fig. 2 and 5, an example of a light emitter 13 is arranged in the distal tip housing 16. The light emitter 13 is arranged to emit light towards an observation target through the light emitter window 14 (see fig. 2). The light emitter window 14 may be separated from the camera window 12 by a non-transparent part of the distal tip to minimize risk of direct light reflections into the camera 11 via one common window in front of both camera 11 and light emitter 13.

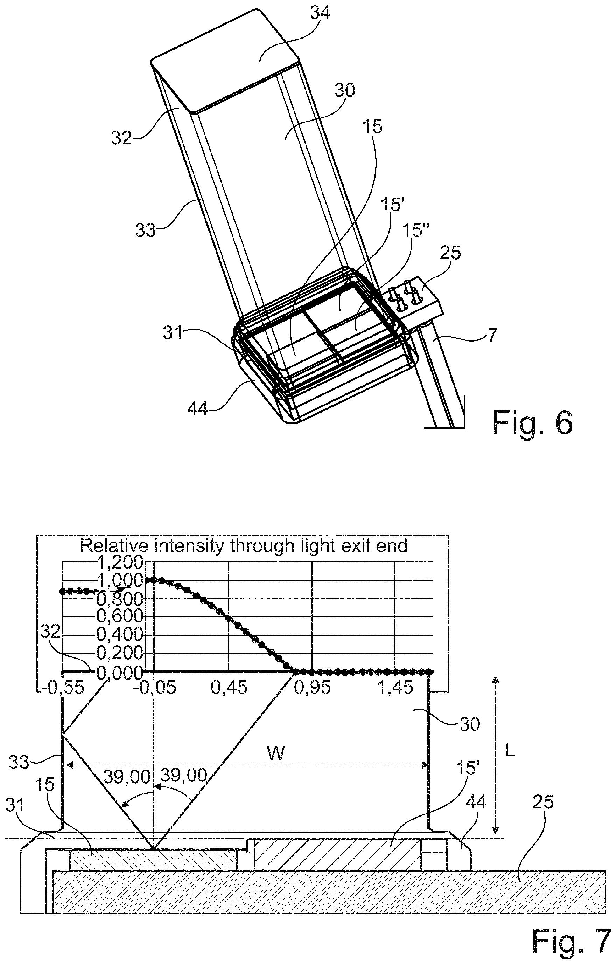

An example of a light emitter 13 is shown in fig. 6. The light emitter in fig. 6 comprises a light guide 30 having a light entry end 31 and a light exit end 32. The light emitter is configured such that light passes through the light guide in the length direction. LEDs 15 are arranged at the light entry end 31 to emit light into the light entry end of the light guide 30. The light entry end 31 may be a plane or a substantially plane surface, and the at least two LEDs 15 may be arranged to abut against this surface with the light emitting part of the LEDs. The number of LEDs will often be two, three or four. The LEDs may emit light having different spectral distributions. At least two LEDs can emit light having different spectral distributions. The LEDs may be connected to a printed circuit board (PCB) 25 through which power is provided to the LEDs. The PCB 25 may be a rigid PCB, enabling simpler alignment of the LEDs during manufacturing, or it may be a flexible PCB, e.g., a flex-print.

Alternatively, the LEDs may be staggered longitudinally to allow an overall reduction in cross-section. This may be possible when the light emitting surface of the LED is smaller than its periphery. Thus, one LED may be positioned distally, and overlapping, the periphery of another, but not its light emitting surface.

Alternatively, rather than using multiple independently packaged LEDs, two or more LEDs can be manufactured in one package, potentially reducing the cross-section of the package relative to multiple packages, and simplifying electrical connections.

The LEDs often have a peak in light intensity at a certain wavelength. In an example an LED, such as a first LED, has a peak of light emitted in the range 405 - 425 nm. In an example an LED, such as a second LED, has a peak of light emitted in the range 520 - 540 nm. Such peaks in the light are advantageous for improving contrast in vascular tissue, as hemoglobin has peaks of light absorbance in narrow bandwidth wavelengths around 415 nm and 530 nm, where the 415 nm light penetrate only the outer surface layer of the tissue, whereas the 530 nm light can penetrate deeper into the tissue. The combination of light from the two wavelength ranges gives an improved contrast of vascular tissue.

A peak in the spectral distribution of light from an LED is here understood as a local maximum in light intensity at a given wavelength. An LED may have only one peak in its spectral distribution of light. But an LED may also have two or three peaks or more.

In examples of the teaching the light emitter comprises a third LED configured to emit light with a third spectral distribution which is different from the first and second spectral distribution. A third LED gives further options for selecting one or more spectral distributions of the light applied during an endoscope procedure. A third LED could have a peak of light emitted in the range 550 - 670 nm. Mixing especially light above 580 nm with the above suggested ranges for the first and/or second LED enables generation of white light with a good visualization of details in tissue. There may also be a fourth LED, having a peak of light in a fourth wavelength range, e.g., in the range 440 - 460 nm, or alternatively a fourth LED could have a broad wave length distribution providing a white light.

The LEDs 15 are provided with power through cables 7 or wires. The cables 7 may extend through the insertion cord 3 to the handle 2 or through the handle to the control unit 42. The LEDs may each have one separate cable for the anode, such that the power to each LED can be individually controlled. The separate anode may be connected to each LED through the PCB 25. The LEDs may have one common cathode cable, such that the number of cables is kept at a minimum. The common cathode may be connected to each LED through the PCB 25. The individually controllable LEDs enable changes in both color of light and intensity of light during a procedure. This may be applied for different light settings, e.g., pre-programmed, which may be selectable by the user, or automatically adjustable based on analysis of obtained images.

It may be preferred to place the LEDs 15 as close to each other as possible in order to facilitate better mixing of the light from the different LEDs. If three or four LEDs are applied, arrangement of the LEDs in a pattern forming a 2x2 matrix may be a way to place them as close as possible. Such a 2x2 matrix can be seen in fig. 2 and 6, where the three LEDs are arranged at the light entry end of the light guide, and in this example, they are arranged in a 2x2 matrix. I.e., there will be one empty spot in the matrix.

The light guide 30 may be provided with a rim 44 at the light entry end (see fig. 6). The rim 44 may encircle the PCB 25 with the LEDs and form an indentation or recess in which the PCB 25 is placed. The shape of the PCB 25 and the shape of the indentation formed by the rim 44, may be configured to fit each other, so that the position of the PCB 25 is aligned in relation to the light guide 30 when the PCB is arranged in the indentation. This facilitates a simple and reliable assembly process for the manufacturing. After placement of the PCB with LEDs, the indentation may be filled with glue to seal and fixate the PCB with LEDs. Part of the PCB may extend outside this sealing and connect to the cables 7 providing power supply to the LEDs.

The light guide 30 may have a rectangular or squared cross-sectional shape when the cross-section is made transversely to a longitudinal axis of the light guide 30, going from a center of the light entry end 31 to a center of the light exit end 32. Fig. 2, 5, 6 illustrates a squared cross-sectional shape. The term center may here be understood as a geometric center. This longitudinal axis may be parallel to an optical axis of the camera. The shape could also take any other form, such as circular or pentagonal or any polygon shape. When the shape is rectangular, or any polygon, the corners may be rounded.

An advantage by the light guide 30 having a rectangular or squared cross-sectional shape, is that most commercially available LEDs have a rectangular or squared shape, and the endoscope image shown to the user via the monitor 41 is also rectangular or squared.

The cross-sectional area of the light guide 30 may be the same from the light entry end 31 to the light exit end 32. Alternatively, the cross-sectional area may be expanding, so that the cross-sectional-area close to the light exit end 32 is larger than the cross-sectional area close to the light entry end 31. This will, as such, have the effect of reducing the spreading of the light, i.e., changing the spatial distribution of emitted light towards a more collimated light beam. However, when a diffusor is added at the light exit end 32, as described below, the diffusor will have the major impact on the spreading of light. One further advantage of the expanding cross-sectional area of the light guide 30, is that if the light guide is integrated with the light emitter window 14, and e.g., molded in one part with this, the demolding will be simpler. The same advantage is present if the light guide is molded as an integrated part of the distal tip housing 16.

A width of the light guide may be defined as the length of the sides when the cross-section of the light guide, transversely to the longitudinal axis, forms a square. If the cross-section is rectangular or any other shape, the width may be the length of the sides of a square having the same cross-sectional area. A length of the light guide may be defined as the distance from a center of the light entry end 31 to a center of the light exit end 32, i.e., the length along a longitudinal axis of the light guide.

In an example, the length of the light guide is equal to the width or larger than the width of the light guide. In one example the length of the light guide is at least a factor of 2 larger than the width. In another example the length is at least three times the width. In another example the length is at least four times the width. As discussed below in relation to fig. 7-9, a longer light guide will result in a better mixing of the light from different LEDs emitting light of different colors.

In an example of the light guide dimensions the width of the light guide 30 may be 1 - 2.5 mm, and the length of the light guide may be 2 - 8 mm.

The distal tip 10 of the endoscope 1 may comprise a light emitter window 14 arranged such that light from the light exit end 32 passes through the light emitter window 14, and where the light emitter window 14 has an inner surface facing the light guide and an oppositely placed external surface. The light guide 30 and the light emitter window 14 may be one integrated part, in which case the inner surface of the light emitter window 14 and the light exit end 32 will be the same plane inside the material forming the integrated light guide 30 and light emitter window 14. This plane may be transverse to the optical direction of the camera 11.

An integrated light guide 30 and light emitter window 14 may be an integrated part of the distal tip housing 16. The three parts may be fused together, e.g., in a molding process. The molding process may be a two component (or two-shot) molding process, such that both the transparent parts, e.g., camera window 12, light emitter window 14, and light guide 30, and the non-transparent parts, e.g., outer wall of distal tip housing 16 different from windows, inner support walls 36, 37, and support structure 38 can be formed in one process.

The light guide 30 may be surrounded by air along its side walls 33, to ensure total internal reflection (TIR) to provide the desired illumination characteristics or light distribution profile from the light source. TIR is based on the characteristics of the materials, which define a critical angle. The critical angle is measured from a plane perpendicular to a transition surface between two mediums, one being the material of the light guide and the other being the material or volume surrounding it. If the angle of incidence of light rays emitted by a light source into the light guide is larger than the critical angle, meaning that rays are closer to being parallel to the transition surface, the rays refracted at the transition surface will not emerge from the medium in which they are transmitted but will be reflected back into the medium. The geometry of the light guides 15 are configured, together with the choice of light source and materials, to substantially reduce the likelihood of a light ray impinging on the transition surface at an angle smaller than the critical angle. The configurations may involve index of refraction of the material, transparency, length, variation of the cross-sectional area, geometry of the cross-section, cladding etc.

Generally, to achieve TIR light has to travel from an optically denser medium (higher refractive index) to an optically less dense medium (lower refractive index). If cladding is used, the cladding has to be optically less dense than the material of the light guide, which is why air works well. Example materials for the light guide include acrylic (poly(methyl methacrylate)) polycarbonate, glass, and other polymers.

The light guide 30 is designed to collect the light emitted from the LEDs. The light rays from one LED 15 will either go straight through the light guide from the light entry end 31 to the light exit end 32 without being reflected at any side walls 33 of the light guide, or it will be reflected by side walls 33 a number of times. The LEDs are not centered on the light entry end 31, therefore, more light rays from a single LED will be reflected by the side wall closest to the LED 15. Depending on the length of the light guide 30 the number of light rays being reflected by side walls will increase and at a certain length the light rays will be reflected on both the closest side wall and on the opposite side wall.

This is illustrated in Fig. 7 - 10 showing how light from one LED 15 may be distributed by light guides having different length/width (L/W) ratios. These figures show cross-sectional views of the light guide 30 in a plane extending in parallel with the longitudinal axis of the light guide. The cross-sectional view passes two LEDs 15, 15' and the PCB 25. The difference between fig. 7, 8, 9 and 10 is the length L of the light guide 30.

In each of fig. 7 - 10 a graph has been placed at the light exit end 32 surface of the light guide 30. This graph illustrates how light intensity from one LED 15 is distributed in this cross-sectional view coinciding with the light exit end 32. I.e., the graphs show the situation where only one LED 15 is powered to emit light. The data in these graphs are based on calculations where the light rays emitted from the LED with the largest angle of scattering are shown. This angle is here 39 degrees as indicated, but the angle may vary depending on the LEDs applied. The light guide 30 is in the examples shown in fig. 7-10 made from polycarbonate. The x-axis of these graphs extends in parallel with the cross-section and perpendicular to the longitudinal axis of the light guide 30. The zero point of the x-axis is aligned with the center of the light emitting area of the active LED 15. The unit for the x-axis is milimeter. The y-axis of the graphs extends in parallel with the longitudinal axis of the light guide 30. The zero point of the y-axis has been placed at the light exit end 32. The shape of each curve illustrates the relative intensity distribution. The numbers indicated on the y-axis are only relative numbers.

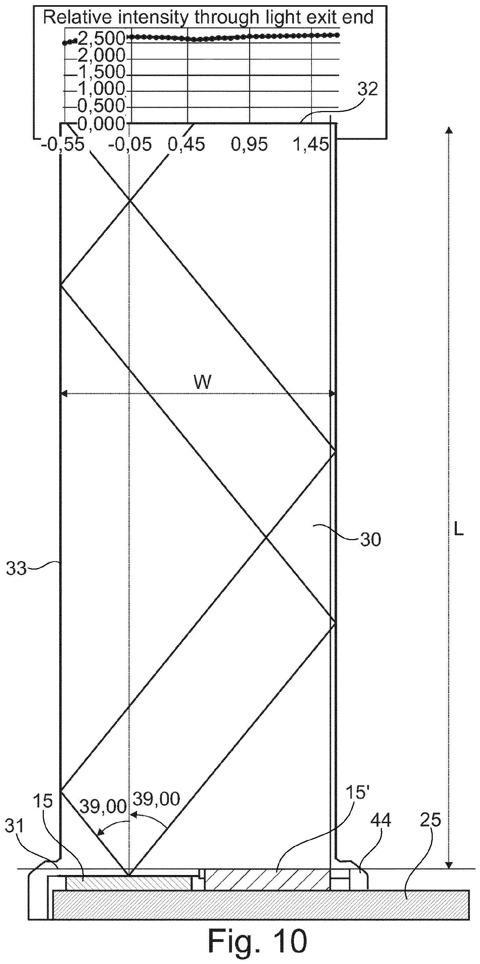

As seen from fig. 7 - 10, the distribution of light intensity from one LED 15 across the light exit end 32, gets more homogeneous with increasing length L of the light guide 30. Simulations and tests have shown that a light guide 30 with a cross section of approximately 2x2mm has an approximately fully homogeneous light intensity distribution when it is at least 6 mm long (i.e., a width W to length L ratio of 1:3). A 6 mm long light guide is shown in fig. 10.

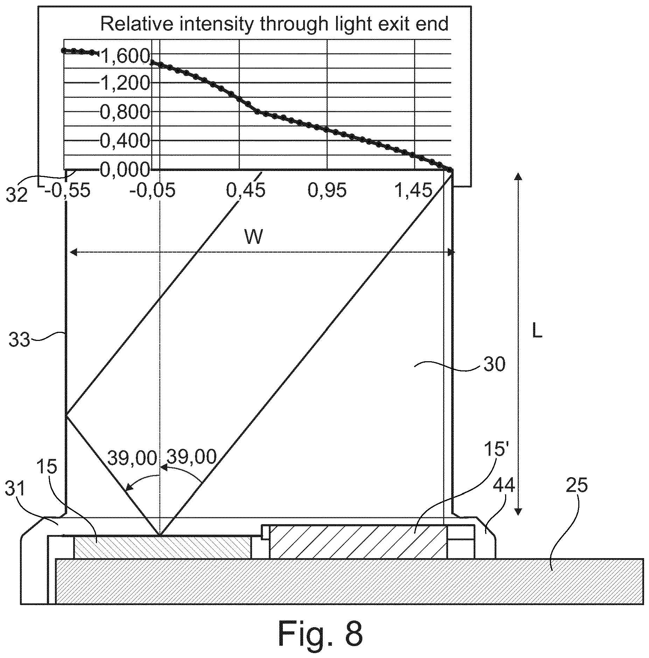

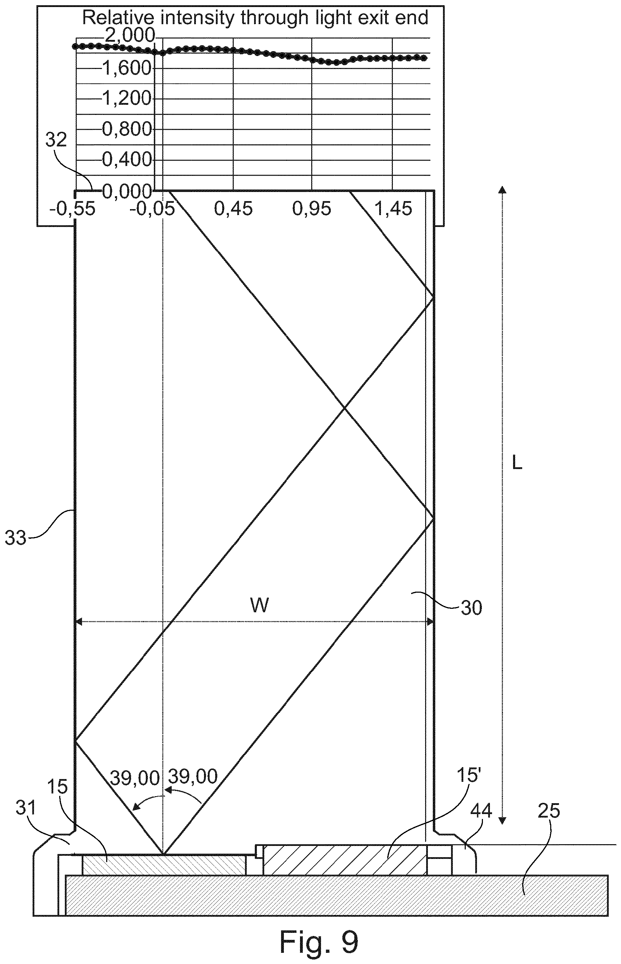

Fig. 9 shows a 4 mm long light guide (ratio 1:2), which provides an almost homogeneous light intensity distribution. Even a 2 mm long light guide 30 may be used in the distal tip 10 of an endoscope 1 even though the light intensity distribution will not be homogeneous (shown in fig. 8). However, if the length of the light guide 30 is only 1 mm, as shown in fig. 7, it is seen that there will be parts of the light exit end 32 which receives a relatively small amount of light intensity from the one powered LED 15.

For any length of light guide 16 limited to fit within the dimensions of a distal tip housing 16 of an endoscope, the off-centered placement of the LEDs 15 in relation to a centered longitudinal axis of the light guide means that more light rays from an LED 15 will be reflected in a nearby side wall 33 of the light guide, and relatively fewer light rays will be reflected in an oppositely placed side wall further away from the LED. This results in a non-symmetric angular distributions of light rays exiting the light exit end 32 of the light guide 30.

This non-symmetric angular distribution of light from each of at least two LEDs 30 having different colors, means that in some angles of the illumination light one color may be more dominant than others. When the illumination light is reflected in tissue during an endoscopic procedure, it may be possible to see colored reflections caused by the non-symmetric distribution. Such reflections of different colors may be disturbing to the endoscope user, even though the use of the light guide will reduce the colored reflections.

It is to be noted that an increasing length of the light guide 30 will reduce the risk of such colored reflections. However, in practice it may be necessary to limit the length of the light guide to maybe 12 mm or less. Often a maximum length may be 15 mm. This limitation may be caused by a preference to have the light guide and the LEDs inside the distal tip housing 16, and not extending proximal of the distal tip housing 16. Thereby, it is simpler to seal off the distal tip housing in a watertight manner. There is an advantage in having the non-bendable stiff part distal to the bending section 20 as short as possible. I.e., the distal stiff part, comprising the distal end bending segment and the distal tip housing and any intermediate connector or transition part, should be as short as possible in a proximal to distal direction, as this will provide the best possible maneuverability of the endoscope 1.

In an example the length of the light guide 30 is at maximum a factor of 8 larger than the width of the light guide, or the length of the light guide 30 is at maximum a factor of 6 larger than the width of the light guide. This will enable for a very good mixing of light in the light guide and will often be short enough to keep the light guide 30 and the LEDs 15 within the distal tip housing 16.

It has been found that adding a diffuser 34 as shown in fig. 6, close to or distal to the light exit end 32, can further reduce or remove the risk of getting colored reflections. A diffusor 34 may be referred to as a light diffusor or an optical diffusor. A diffusor has the effect of scattering the light. A diffuser may here be a transmissive diffuser, i.e., diffusing light passing through the diffusor. A diffuser 34 may be a randomized or stochastic microstructure which scatters all incoming light rays. The diffusor 34 may be a white diffusing glass or polymer made from a white translucent material. The diffusor 34 may, alternatively, be a transparent material provided with a microstructure or nanostructure surface. The transparent material may be a glass or a polymer, and the microstructure surface may be provided as a ground surface by sandblasting, or as part of the molding process. The diffusor may, alternatively, be made by a holographic etching on a transparent material. A diffusor may also be a combination of these alternatives and may also be made in other ways.

The diffusor 34 may scatter light transmitted through the diffusor in a Lambertian scattering pattern. In Lambertian scattering the radiance of the scattered light is independent of the angle of view. The diffusor 34 may also scatter light to achieve a gaussian light intensity distribution. Also, other light intensity distributions on the distal side of the diffusor are possible.

The diffuser 34 may be a separate component, e.g., in the form of a film, or a diffusing pattern imprinted or molded at either the light exit end 32 of the light guide or at an inner or outer surface of the light emitter window 14. The diffuser 34 may also be a layer of particles or microstructure, e.g., made by a laser, within the light guide material at or close to the light exit end 32, or within the light emitter window 14.

In one example the diffusor 34 is provided at a surface of the window or at the light exit end. In a further example, the light guide and the light emitter window 14 is one integrated part e.g., molded in one process or fused together, and the diffusor is provided at the external surface of the light emitter window 14. This may be by an imprinted or a molded pattern with a light diffusing effect on the external surface. Both examples reduce the number of components to be provided and assembled. Therefore, manufacturing cost can be reduced.

It has been found in experiments that the combination of the one light guide 30 and the diffuser 34 makes it possible to avoid the colored reflections into the camera of an endoscope having at least two LEDs of different colors placed in the distal tip for illumination of a target to be observed.

The diffuser 34 may be designed to have a scattering angle corresponding to or being larger than the field of view of the camera 11. The scattering angle may be defined by the angle at which the light intensity is half the maximum light intensity. The maximum light intensity is often in the direction extending in parallel to the optical axis OA of the camera 11. The scattering angle may also be designed according to the intended use of the endoscope 1. Thereby, utilizing the light emitted from the LEDs in the most optimal way. A bronchoscope is mostly used in a tube-like structure and a relatively small scattering angle may be preferred. A cystoscope is mostly used in a spherical cavity and a relatively large scattering angle may be preferred. The scattering angle of a diffusor may be adjusted by varying particle size, or haze or other parameters used to obtain the diffusive effect.

The diffuser 34 may be designed to cover the light exit end 32 of the light guide 30. If the light emitter window 14 is between the diffuser 34 and the light exit end 32, e.g., when the diffusor is on the external side of the light emitter window 14, the diffusor may be slightly larger than the light exit end of the light guide. Thereby, it may be avoided that light exiting the light exit end 32 close to the edge between the light exit end 32 and the side walls 33 of the light guide 30, can get around and bypass the diffusor 34.

### List of references

| | | | |
|---|---|---|---|
| 1 | endoscope | 25 | Printed Circuit Board for LEDs (PCB) |
| 2 | handle | 26 | distal working channel opening |
| 3 | insertion cord | 27 | segment |
| 4 | electrical cable with plug | 28 | hinge |
| 5 | main tube | 30 | Light guide |
| 6 | working channel | 31 | light entry end |
| 7 | electrical wires | 32 | light exit end |
| 10 | distal tip | 33 | side wall |
| 11 | camera | 34 | diffusor |
| 12 | camera window | 36 | support wall working channel tube |
| 13 | light emitter | 37 | support wall media tubes |
| 14 | light emitter window | 38 | support structure for camera |
| 15 | first LED | 40 | screen |
| 15' | second LED | 41 | monitor |
| 15" | third LED | 42 | control unit |
| 16 | distal tip house | 43 | visualization system |
| 17 | image sensor | 44 | rim |
| 18 | lens stack | 46 | bending lever |
| 19 | flexible printed circuit board | OA | optical axis |
| 20 | bending section | L | length of light guide |
| 21 | cleaning nozzle | W | width of light guide |
| 22 | waterjet nozzle | | |

## Claims

1. An endoscope comprising a distal tip, including a camera with an optical axis, and a light emitter, the light emitter comprising:
- a first light emitting diode (LED) configured to emit light with a first spectral distribution,
- a second LED configured to emit light with a second spectral distribution which is different from the first spectral distribution,
- a light guide with a length, and a width transverse to the length, the light guide comprising a light entry end and a light exit end configured such that light passes the light guide in the length direction, and
- a diffusor arranged transverse to the optical axis;
wherein the first and the second LED are arranged to emit light into the light entry end, and the diffusor is arranged such that light passing the light exit end also passes the diffusor.

2. The endoscope according to claim 1, wherein the length of the light guide is equal to, or larger than the width of the light guide, or the length of the light guide is at least a factor of 2 larger than the width.

3. The endoscope according to claim 1 or 2, wherein the distal tip comprises a light emitter window arranged such that light from the light exit end passes through the light emitter window, and where the light emitter window has an inner surface facing the light guide and an oppositely placed external surface.

4. The endoscope according to claim 3, wherein the diffusor is provided at the inner surface or the external surface of the light emitter window or at the light exit end of the light guide.

5. The endoscope according to claim 3 or 4, wherein the light guide and the light emitter window is one integrated part, where the light guide extends from the inner surface of the window, and the diffusor is placed at the external surface of the light emitter window.

6. The endoscope according to any one of the previous claims, wherein the light emitter comprises a third LED configured to emit light with a third spectral distribution which is different from the first and second spectral distribution.

7. The endoscope according to claim 6, wherein the light emitter comprises a fourth LED configured to emit light with a fourth spectral distribution which is different from the first, second and third spectral distributions.

8. The endoscope according to claim 6 or 7, wherein the LEDs are arranged at the light entry end of the light guide in a 2x2 matrix.

9. The endoscope according to any one of the previous claims, wherein the first LED has a peak of light emitted in the range 405 - 425 nm.

10. The endoscope according to any one of the previous claims, wherein the second LED has a peak of light emitted in the range 520 - 540 nm.

11. The endoscope according to any one of the previous claims, wherein the second LED, or the third LED according to claim 6, has a peak of light emitted in the range 550-670 nm.

12. The endoscope according to any one of the previous claims, wherein the length of the light guide is at maximum a factor of 8 larger than the width, or the length of the light guide is at maximum a factor of 6 larger than the width.

13. The endoscope according to any one of the previous claims, wherein the power supply to at least the first and the second LEDs, is controllable for each LED separately, such that light intensity from each of at least the first and the second LEDs can be adjusted independently.

14. The endoscope according to any one of the previous claims, wherein the distal tip comprises a housing with a support for positioning the camera, where the support is non-transparent to prevent stray-light into the image sensor.

15. A system comprising an endoscope according to any one of claims 1-14, a monitor and a control unit.
